# EUROPEAN PATENT SPECIFICATION

(11) **EP 1 016 698 B1**
(45) Date of publication and mention of the grant of the patent: **22.09.2004**
(21) Application number: 00104425.4
(22) Date of filing: 13.06.1994
(51) Int. Cl.: C09D 7/00, C09J 11/06, C09D 11/02

(54) **Use of a solvent composition containing an oxyisobutyric acid ester as a solvent in coatings, adhesives and printing inks**
Verwendung einer Lösungsmittel-Zusammensetzung enthaltend ein Oxyisobuttersäureester als Lösemittel in Beschichtungen, Klebstoffen und Druckfarben
Utilisation d'une composition de solvant contenant un ester d'acide oxyisobutyrique comme solvant dans les revêtements, les adhésifs et les encres

(30) Priority: 15.06.1993 JP 16737493; 22.06.1993 JP 17360693; 17.09.1993 JP 25368893; 24.12.1993 JP 34605693; 11.03.1994 JP 10345794
(43) Date of publication of application: 05.07.2000
(62) Divisional of application: 94109036.7
(73) Proprietor: Mitsubishi Rayon Co., Ltd., Tokyo 108-8506 (JP)
(72) Inventor: Takayanagi, Yasuyuki, Nitto Chem. Ind. Co., Ltd., Yokohama-shi, Kanagawa (JP); Endou, Satoshi, Nitto Chem. Ind. Co., Ltd., Yokohama-shi, Kanagawa (JP); Sugama, Naoki, Nitto Chem. Ind. Co., Ltd., Yokohama-shi, Kanagawa (JP)
(74) Representative: HOFFMANN - EITLE

(56) References cited:
- PATENT ABSTRACTS OF JAPAN vol. 016, no. 110 (C-0920), 18 March 1992 (1992-03-18) & JP 03 284651 A (SHOWA DENKO KK;OTHERS: 01), 16 December 1991 (1991-12-16)
- PATENT ABSTRACTS OF JAPAN vol. 009, no. 218 (C-301), 5 September 1985 (1985-09-05) & JP 60 078937 A (MITSUBISHI GAS KAGAKU KK), 4 May 1985 (1985-05-04)

## Description

### FIELD OF THE INVENTION

This invention relates to a solvent composition, and more particularly to a solvent composition suitable for use as a solvent in coatings, adhesives, and printing inks.

### BACKGROUND OF THE INVENTION

Solvents have played an important roll in the development of the chemical industry. In particular, the importance of solvents in the fields of coating compositions, adhesives and printing inks has been ever increasing, with the recent remarkable development in the plastics industry. Coating compositions, adhesives, printing inks, etc. are generally used in the form of a solution in a solvent for assuring ease in handling on use, and for uniformly and intimately applying a high polymer base to a substrate. To this effect, it is very important for the solvent to have moderate volatility, while retaining sufficient dissolving power for the high polymer base and uniform coating properties, as well as ease in handling. In other words, the quality of coating compositions, adhesives, and printing inks largely depends on the choice of solvent.

Glycol ether type cellosolves, especially Cellosolve acetate (ethylene glycol monoethyl ether acetate), have been used for their excellent properties as solvents for cellulose resins, epoxy resins, acrylic resins, vinyl resins (e.g., vinyl acetate resins and vinyl chloride resins), alkyd resins, and polyester resins which are commonly used in the fields of coating compositions, adhesives and printing inks. In recent years, however, demand for safety of chemical substances has been increasing from the standpoint of environmental pollution. In this regard, use of Cellosolve acetate is strictly limited because of its toxicity, and the Japanese Industrial Safety and Health Law laid down criteria for controlling the working environment concentration thereof.

Intensive studies have therefore been directed to development of a solvent which can be a satisfactory substitute for Cellosolve acetate in terms of dissolving power, and yet gives rise to no safety problem. For example, ethyl lactate, propylene glycol monomethyl ether acetate, methoxypropanol, and ethyl β-ethoxypropionate have been studied as promising alternatives. However, they are not always satisfactory in dissolving power, safety, smell, and ease in handling. Of these alternative solvents, ethyl lactate, which is permitted as a food additive, seems the most preferred from the standpoint of safety, but it is not deemed to have sufficient dissolving power for high polymers and various additives. While alkyl β-alkoxypropionates, such as methyl β-methoxypropionate and ethyl β-ethoxypropionate, appear to be the most preferred from the viewpoint of dissolving power, they are still unsatisfactory in terms of dissolving power for high polymers or various additives, and in volatility after application. A mixture of methyl β-methoxypropionate and ethyl β-ethoxypropionate has been proposed as a solvent with improved physical properties, as disclosed in JP-A-3-284651 (the term "JP-A" as used herein means an "unexamined published Japanese patent application"). However, preparation of mixture involves a complicated operation, and is not suitable for industrial use. Thus, a practical solvent equal to Cellosolve acetate in performance has not yet been developed.

### SUMMARY OF THE INVENTION

An object of the present invention is to provide a solvent composition freed of the drawbacks of conventional solvents, such as Cellosolve acetate, comprising a solvent system which is of low toxicity and harmless to humans, has high dissolving power, does not produce environment destructive substances, does not give off offensive odor, and has a relatively high boiling point indicative of safety and ease in handling.

As a result of extensive research into a solvent composition having the above-described favorable properties, the present inventors have found that the object of the present invention can be met by an oxyisobutyric acid ester selected from an alkyl α-alkoxyisobutyrate, alkyl β-alkoxyisobutyrate, and alkyl α-hydroxyisobutyrate. The present invention has been completed based on this finding.

The present invention provides the use of a solvent composition containing, as an active component, in an amount of at least 5% by weight at least one oxyisobutyric acid ester selected from the group consisting of an alkyl α-alkoxyisobutyrate represented by formula (I): 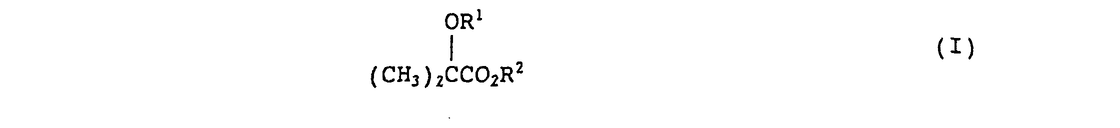 an alkyl β-alkoxyisobutyrate represented by formula (II): 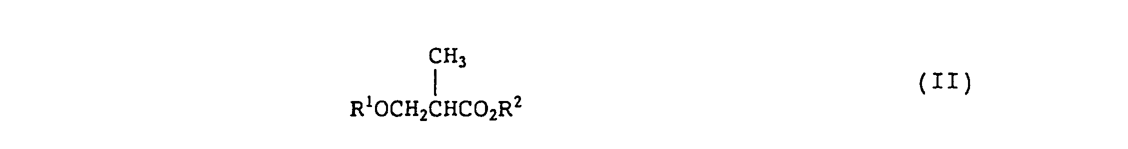 and an alkyl α-hydroxyisobutyrate represented by formula (III): 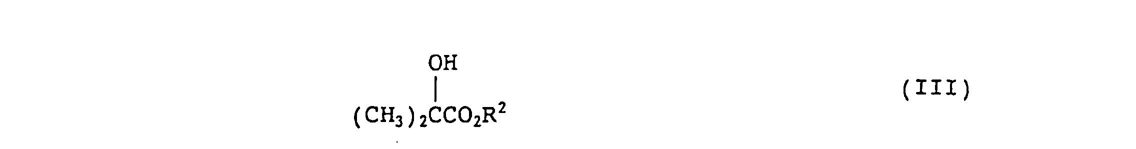 wherein R¹ and R² each represent an alkyl group having from 1 to 4 carbon atoms, as a solvent in coatings, adhesives and printing inks.

### DETAILED DESCRIPTION OF THE INVENTION

The oxyisobutyric acid esters represented by formulae (I), (II) and (III), which can be used in the present invention, are available as disclosed, for example, in EP-A-429800.

The solvent composition of the present invention essentially contains an alkyl oxyisobutyrate. The alkyl oxyisobutyrates include alkyl α-alkoxyisobutyrates (I), such as methyl α-methoxyisobutyrate, ethyl α-methoxyisobutyrate, methyl α-ethoxyisobutyrate, and ethyl α-ethoxyisobutyrate; alkyl β-alkoxyisobutyrates (II), such as methyl β-methoxyisobutyrate, ethyl β-methoxyisobutyrate, methyl β-ethoxyisobutyrate, ethyl β-ethoxyisobutyrate, methyl β-isopropoxyisobutyrate, ethyl β-isopropoxyisobutyrate, butyl β-isopropoxyisobutyrate, methyl β-butoxyisobutyrate, ethyl β-butoxyisobutyrate, and butyl β-butoxyisobutyrate; and alkyl α-hydroxyisobutyrates (III), such as methyl α-hydroxyisobutyrate, ethyl α-hydroxyisobutyrate, isopropyl α-hydroxyisobutyrate, and butyl α-hydroxyisobutyrate. From the standpoint of dissolving power and volatility, methyl α-methoxyisobutyrate, ethyl α-ethoxyisobutyrate, methyl β-methoxyisobutyrate, ethyl β-methoxyisobutyrate, methyl β-ethoxyisobutyrate, ethyl β-ethoxyisobutyrate, methyl β-isopropoxyisobutyrate, methyl β-butoxyisobutyrate, ethyl β-butoxyisobutyrate, butyl β-butoxyisobutyrate, methyl α-hydroxyisobutyrate, ethyl α-hydroxyisobutyrate, isopropyl α-hydroxyisobutyrate, and butyl α-hydroxyisobutyrate, are preferred.

The alkyl α-alkoxyisobutyrates represented by formula (I), alkyl β-alkoxyisobutyrates represented by formula (II) and alkyl α-hydroxyisobutyrates represented by formula (III) may be used either individually or in combination of two or more thereof. While not limiting in combination, alkyl oxyisobutyrates represented by formula (I) or (II) is preferably used in combination with α-hydroxyisobutyrates represented by formula (III), in order to take advantage of dissolving power. The proportion of the alkyl oxyisobutyrates represented by formula (I) or (II)/α-hydroxyisobutyrates represented by formula (III) is preferably from 5/95 to 95/5 by weight, more preferably from 10/90 to 90/10 by weight, most preferably from 30/70 to 70/30 by weight.

The alkyl α-alkoxyisobutyrates, alkyl β-alkoxyisobutyrates, and alkyl α-hydroxyisobutyrates are compatible with other general organic solvents, such as alcohols, esters, ketones, amides, and aromatic hydrocarbons. They exhibit markedly excellent dissolving power for a wide range of high polymers, which include natural resins, such as cellulose resins, and synthetic resins, such as epoxy resins, acrylic resins, vinyl resins (e.g., vinyl acetate resins and vinyl chloride resins), alkyd resins, polyester resins, and novolak resins, as well as general hydrocarbon-based fats and oils. Accordingly, the solvent composition comprising, as an active compound, the oxyisobutyric acid esters represented by formulae (I), (II) and (III) in an amount of at least 5 % by weight can be used as a solvent in coatings, adhesives and printing inks which comprise at least one of the following components: a cellulose resin, epoxy resin, acrylic resin, vinyl resin, alkyd resin, polyester resin, a polystyrene, a methyl methacrylate-styrene copolymer, an acrylonitrile-styrene copolymer, a phenoxy resin, a polysulfone. The oxyisobutyric esters of the present invention not only serve as a solvent by themselves, but they also exhibit excellent performance as a diluent or an auxiliary solvent for other organic solvents. Thus, they can be formulated into a solvent composition together with other organic solvents. The proportion of the oxyisobutyric ester in the solvent composition is not less than 5% by weight, and preferably not less than 10% by weight, in order to take full advantage of the safety and dissolving power of the oxyisobutyric ester.

The other solvents with which the oxyisobutyric esters of the present invention may be combined are not particularly limited and include water, alcohols, ethers, esters, ketones, amides, aliphatic hydrocarbons, and aromatic hydrocarbons. Suitable examples of the other solvents are water, methanol, ethanol, isopropyl alcohol, butanol, methyl isobutyl carbinol, heptanol, octanol, nonanol, 3-methylbutanol, propylene glycol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, pyrrolidone, N-methylpyrrolidone, dimethylformamide, dimethylacetamide, dimethyl sulfoxide, sulfolane, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, nitromethane, nitroethane, acetonitrile, γ-butyrolactone, propylene glycol monomethyl ether acetate, toluene, xylene, hexane, and cyclohexane. Preferred of them are water, methanol, ethanol, isopropyl alcohol, butanol, heptanol, octanol, 3-methylbutanol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, N-methylpyrrolidone, dimethylformamide, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, acetonitrile, propylene glycol monomethyl ether acetate, toluene, and xylene.

These organic solvents and water may be used either individually or in combination of two or more thereof. A combined use of the organic solvent or water makes it possible to appropriately improve or modify safety and ease in handling of the solvent composition of the present invention.

Since the alkyl oxyisobutyrates of the present invention have a high boiling point and a relatively low rate of evaporation, they are useful as high-boiling solvents. When compounded into mixed solvent systems, they bring about improvements in performance properties and workability of coating compositions, adhesives, and ink compositions, such as spreadability and smoothness of a coating film and effects on fusion of resins.

The content of the alkyl oxyisobutyrate in the solvent composition is not less than 5% by weight, and preferably not less than 10% by weight, depending on the use.

The oxyisobutyric esters of the present invention have very high dissolving power for various organic compounds, including high polymers and naturally-occurring compounds. On the other hand, the alkyl oxyisobutyrates of the present invention do not have risk of explosion at an ordinal temperature, because their ignition point is 40°C or more. In addition, the acute toxicity (median lethal dose (LD₅₀); rat, oral) of the alkyl oxyisobutyrates is 2000mg/kg or more. Therefore, they are also useful as an assistant for diluting solvents for agricultural chemicals or as a solvent or an assistant for cosmetics.

The present invention will now be illustrated in greater detail with reference to the Examples in view of the Comparative Examples, but the present invention should not be construed as being limited thereto. All of the mixing ratios of the solvents are given by weight unless otherwise indicated.

### EXAMPLE 1

The compatibility of the alkyl oxyisobutyrates of the present invention with other solvents was examined as follows.

Methyl α-methoxyisobutyrate, methyl β-methoxyisobutyrate, methyl α-hydroxyisobutyrate or a 30/70 mixture of methyl β-methoxyisobutyrate and methyl α-hydroxyisobutyrate, and the solvents shown in Table 1 below were mixed in a mixing ratio of 1/1 by volume in an Erlenmeyer flask, and the mixture was allowed to stand still at room temperature. The degree of compatibility was visually observed and rated as follows. The results obtained are shown in Table 1.
A ... Completely uniformly mixed.
B ... White turbidity observed.
C ... Separated into two phases.

### COMPARATIVE EXAMPLE 1

The compatibility of Cellosolve acetate (ethylene glycol monoethyl ether acetate; hereinafter abbreviated as "ECA") with the solvent shown in Table 1 was examined in the same manner as in Example 1. The results obtained are shown in Table 1 below.

**TABLE 1**

| Solvent | Example 1 | | | | Compar. Example 1 |
|---|---|---|---|---|---|
| | α-MBM¹⁾ | β-MBM²⁾ | α-HBM³⁾ | β-MBM/ α-HBM⁴⁾ | ECA |
| Water | C | C | A | B | C |
| Methanol | A | A | A | A | A |
| Ethanol | A | A | A | A | A |
| Isopropyl alcohol | A | A | A | A | A |
| Ethyl acetate | A | A | A | A | A |
| Acetone | A | A | A | A | A |
| dimethylformamide | A | A | A | A | A |
| Acetonitrile | A | A | A | A | A |
| Benzene | A | A | A | A | A |
| Toluene | A | A | A | A | A |
| Xylene | A | A | A | A | A |
| n-Hexane | A | A | A | A | A |
| Cyclohexane | A | A | A | A | A |
| Cyclohexanone | A | A | A | A | A |
| Cyclohexanol | A | A | A | A | A |

| | | | | | |
|---|---|---|---|---|---|
| Note: 1): Methyl α-methoxyisobutyrate | | | | | |
| 2): Methyl β-methoxyisobutyrate | | | | | |
| 3): Methyl α-hydroxyisobutyrate | | | | | |
| 4): β-MBM/α-HBM = 30/70 | | | | | |

### EXAMPLE 2

The rate of evaporation of the alkyl oxyisobutyrates of the present invention was measured as follows.

The solvents shown in Table 2 below weighing 0.75 g, were uniformly spread on a disc of No. 4 filter paper having a diameter of 95 mm. The coated filter paper was allowed to stand on a testing stand at 25°C with a covering over it. The sample was weighed at given time intervals to determine the evaporation loss, from which the rate of evaporation was calculated. The results obtained, expressed relatively taking the rate of evaporation of butyl acetate as a standard (100), are shown in Table 2 below.

**TABLE 2**

| Solvent | Relative Rate of Evaporation |
|---|---|
| Butyl acetate | 100 (standard) |
| Methyl α-hydroxyisobutyrate | 56 |
| Ethyl α-Hydroxyisobutyrate | 26 |
| Methyl α-methoxyisobutyrate | 64 |
| Methyl β-methoxyisobutyrate | 44 |
| Ethyl β-ethoxyisobutyrate | 16 |

### COMPARATIVE EXAMPLE 2

The rate of evaporation of the conventional solvents shown in Table 3 below was determined in the same manner as in Example 2. The results obtained are shown in Table 3 below.

**TABLE 3**

| Solvent | Relative Rate of Evaporation |
|---|---|
| Butyl acetate | 100 (standard) |
| ECA | 22 |
| Ethyl lactate | 23 |
| Methyl β-methoxypropionate | 35 |

### EXAMPLE 3

The dissolving power of methyl β-methoxyisobutyrate for various resins was evaluated as follows.

In a 100 mℓ-volume Erlenmeyer flask was put 2.0 g of the resins shown in Table 4 below, and 20 mℓ of methyl β-methoxyisobutyrate was added thereto. The mixture was kept at 25°C while stirring, and the time required for the resin to dissolve was measured. The results obtained are shown in Table 4 below.

### COMPARATIVE EXAMPLE 3

The dissolving power of ECA was evaluated in the same manner as in Example 3. The results are shown in Table 4 below.

**TABLE 4**

| Resin | Time for Dissolution (min) | |
|---|---|---|
| | Example 3 | Compar. Example 3 |
| Polyester (ER-1002, a product of Mitsubishi Rayon Co., Ltd.) | 110 | 150 |
| Polystyrene (QPX2B, a product of Denki Kagaku Kogyo K.K. | 46 | 86 |
| Methyl methacrylate-styrene copolymer (MS-300, a product of Nippon Steel Chemical Co., Ltd.) | 82 | 125 |
| Acrylonitrile-styrene copolymer (AS-30, a product of Asahi Chemical Industry Co., Ltd.) | 95 | 145 |
| Acrylic resin (Acrydic A-405, a product of Dainippon Ink and Chemicals, Inc.) | 30 | 45 |
| Phenoxy resin (PKH-H, a product of Union Carbide Corp.) | 110 | 150 |
| Polysulfone (P1800NT11, a product of Amoco Co.) | 160 | 260 |

### EXAMPLE 4

The dissolving power of the alkyl oxyisobutyrates shown in Table 5 below for an epoxy resin was evaluated.

In a 100 mℓ-volume Erlenmeyer flask was put 2.0 g of an epoxy resin (Epikote 1007, a product of Yuka Shell Epoxy Co., Ltd.), and 20 mℓ of the alkyl oxyisobutyrate was added thereto. The mixture was kept at 25°C while stirring, and the time required for the resin to dissolve was measured. The results obtained are shown in Table 5 below.

**TABLE 5**

| Solvent | Time of Dissolution (min) |
|---|---|
| Methyl α-methoxyisobutyrate | 22 |
| Methyl β-methoxyisobutyrate | 25 |
| Ethyl β-ethoxyisobutyrate | 28 |
| Methyl α-methoxyisobutyrate/methyl α-hydroxyisobutyrate (50/50) | 29 |
| Methyl α-hydroxyisobutyrate | 38 |

### COMPARATIVE EXAMPLE 4

The epoxy resin-dissolving power of ECA, ethyl lactate or methyl β-methoxypropionate was evaluated in the same manner as in Example 4. The results are shown in Table 6 below.

**TABLE 6**

| Solvent | Time of Dissolution (min) |
|---|---|
| ECA | 45 |
| Ethyl lactate | 65 |
| Methyl β-methoxypropionate | 42 |

As can be seen from Table 1, the oxyisobutyric esters of the present invention have compatibility equal or superior to the conventional general-purpose solvents, such as ECA, and thus can be used as a mixture with a broad range of other solvents.

As can be seen from Tables 2 and 3, the a rate of evaporation can arbitrarily be selected by a proper choice of the ester group. The rate of evaporation of the oxyisobutyric esters of the present invention is about 2 to 3 times as high as that of ECA or ethyl lactate, which have been widely used, proving that the oxyisobutyric esters provide a solvent system having good volatility.

As can be seen from Tables 4, 5 and 6, the oxyisobutyric esters of the present invention need a shorter time for dissolving various resins, often used in coating compositions and adhesives, than needed by the conventional general-purpose solvents. This reveals their excellent resin-dissolving power.

As described above, the solvent composition according to the present invention has excellent dissolving power, gives off no offensive smell, gives rise to no environmental problem, and is safe. Additionally, the solvent composition of the present invention has the following advantages:
1) It has an extremely high dissolving power for natural and synthetic high polymers;
2) It is freely miscible with many organic solvents;
3) It is biodegradable, non-accumulating in nature;
4) It has low toxicity, no teratogenicity, and high safety;
5) It has a relatively high boiling point and ignition point, so as to secure improved handling properties and safety; and
6) It is non-corrosive for a substrate.

## Claims

1. The use of a solvent composition comprising, as an active compound, at least one oxyisobutyric acid ester in an amount of at least 5 % by weight, wherein said oxyisobutyric acid ester is selected from the group consisting of an alkyl α-alkoxyisobutyrate represented by formula (I): 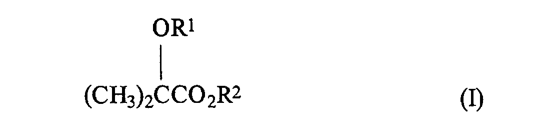 an alkyl β-alkoxyisobutyrate represented by formula (II): 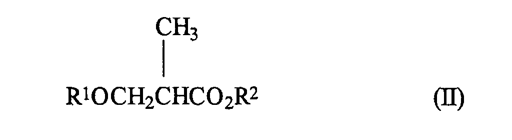 and an alkyl α-hydroxyisobutyrate represented by formula (III): 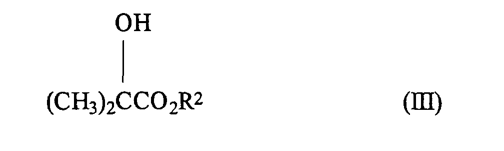 wherein R¹ and R² each represent an alkyl group having from 1 to 4 carbon atoms, as a solvent in coatings, adhesives and printing inks.

2. The use as claimed in claim 1, wherein the coatings, adhesives and printing inks comprise at least one of the following components: a cellulose resin, epoxy resin, acrylic resin, vinyl resin, alkyd resin, polyester resin, a polystyrene, a methyl methacrylate-styrene copolymer, an acrylonitrile-styrene copolymer, a phenoxy resin, a polysulfone.

3. The use as claimed in claim 1 or 2, wherein said solvent composition further comprises at least one compound selected from the group consisting of water, methanol, ethanol, isopropyl alcohol, butanol, methyl isobutyl carbinol, heptanol, octanol, nonanol, 3-methylbutanol, proylene glycol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, pyrrolidone, N-methylpyrrolidone, dimethylformamide, dimethylacetamide, dimethyl sulfoxide, sulfolane, methyl acetate, ethyl acetate, butyl acetate, amyl acetate, 3-methoxybutyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, nitromethane, nitroethane, acetonitrile, γ-butyrolactone, propylene glycol monomethyl ether acetate, toluene, xylene, hexane, and cyclohexane.

4. The use as claimed in any of claims 1 to 3, wherein said oxyisobutyric acid ester is selected from the group consisting of methyl α-methoxyisobutyrate, ethyl α-methoxyisobutyrate, methyl α-ethoxyisobutyrate, ethyl α-ethoxyisobutyrate, methyl β-methoxyisobutyrate, ethyl β-methoxisobutyrate, methyl β-ethoxyisobutyrate, ethyl β-ethoxyisobutyrate, methyl β-isopropoxyisobutyrate, ethyl β-isopropoxyisobutyrate, butyl β-isopropoxyisobutyrate, methyl β-butoxyisobutyrate, ethyl β-butoxyisobutyrate, butyl β-butoxyisobutyrate, methyl α-hydroxyisobutyrate, ethyl α-hydroxyisobutyrate, isopropyl α-hydroxyisobutyrate, and butyl α-hydroxyisobutyrate.

## Patentansprüche

1. Verwendung einer Lösungsmittelzusammensetzung, umfassend als Wirkkomponente wenigstens einen Oxyisobuttersäureester in einer Menge von wenigstens 5 Gew.%, wobei der Oxyisobuttersäureester ausgewählt ist aus der Gruppe, bestehend aus einem Alkyl-α-alkoxyisobutyrat, dargestellt durch Formel (I): 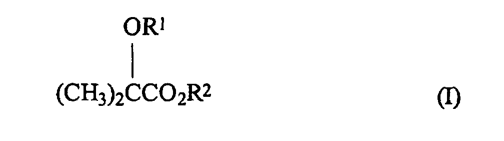 einem Alkyl-β-alkoxyisobutyrat, dargestellt durch Formel (II) : 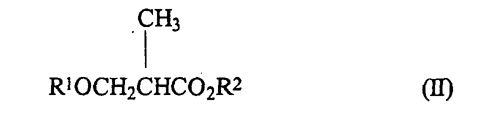 und einem Alkyl-α-hydroxyisobutyrat, dargestellt durch Formel (III): 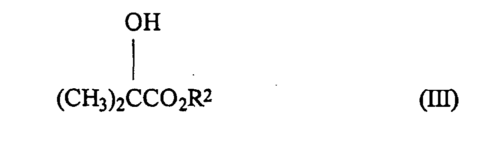 worin R¹ und R² jeweils eine Alkylgruppe mit 1 bis 4 Kohlenstoffatomen darstellen,
als ein Lösungsmittel in Beschichtungen, Haftmitteln und Druckfarben.

2. Verwendung wie in Anspruch 1 beansprucht, worin die Beschichtungen, Haftmittel und Druckfarben wenigstens eine der folgenden Komponenten umfassen: ein Celluloseharz, Epoxyharz, Acrylharz, Vinylharz, Alkydharz, Polyesterharz, ein Polystyrol, ein Methylmethacrylat-Styrol-Copolymer, ein Acrylonitril-Styrol-Copolymer, ein Phenoxyharz, ein Polysulfon.

3. Verwendung wie in Anspruch 1 oder 2 beansprucht, worin die Lösungsmittelzusammensetzung ferner wenigstens eine Verbindung umfaßt, ausgewählt aus der Gruppe bestehend aus Wasser, Methanol, Ethanol, Isopropylalkohol, Butanol, Methylisobutylcarbinol, Heptanol, Octanol, Nonanol, 3-Methylbutanol, Propylenglycol, 3-Methoxybutanol, 3-Methyl-3-methoxybutanol, 3,5,5-Trimethyl-1-hexanol, 2-Ethyl-1-hexanol, Cyclohexanol, Benzylalkohol, Aceton, Methylethylketon, Methylisobutylketon, Cyclohexanon, Isophoron, Pyrrolidon, N-Methylpyrrolidon, Dimethylformamid, Dimethylacetamid, Dimethylsulfoxid, Sulfolan, Methylacetat, Ethylacetat, Butylacetat, Amylacetat, 3-Methoxybutylacetat, Butylacetat, Amylacetat, 3-Methoxybutylacetat, 3-Methyl-3-methoxybutylacetat, Methyllactat, Ethyllactat, Butyllactat, Methyl-3-methoxypropionat, Ethyl-3-ethoxypropionat, 2-Ethylhexylacetat, Cyclohexylacetat, Benzylacetat, Dibenzylether, Nitromethan, Nitroethan, Acetonitril, γ-Butyrolacton, Propylenglycolmonomethyletheracetat, Toluol, Xylol, Hexan und Cyclohexan.

4. Verwendung wie in einem der Ansprüche 1 bis 3 beansprucht, worin der Oxyisobuttersäureester ausgewählt ist aus der Gruppe bestehend aus Methyl-α-methoxyisobutyrat, Ethyl-α-methoxyisobutyrat, Methyl-α-ethoxyisobutyrat, Ethyl-α-ethoxyisobutyrat, Methyl-β-methoxyisobutyrat, Ethyl-β-methoxisobutyrat, Methyl-β-ethoxyisobutyrat, Ethyl-β-ethoxyisobutyrat, Methyl-β-isopropoxyisobutyrat, Ethyl-β-isopropoxyisobutyrat, Butyl-β-isopropoxyisobutyrat, Methyl-β-butoxyisobutyrat, Ethyl-β-butoxyisobutyrat, Butyl-β-butoxyisobutyrat, Methyl-α-hydroxyisobutyrat, Ethyl-α-hydroxyisobutyrat, Isopropyl-α-hydroxyisobutyrat und Butyl-α-hydroxyisobutyrat.

## Revendications

1. Utilisation d'une composition de solvant comprenant comme composé actif au moins un ester d'acide oxyisobutyrique dans une quantité d'au moins 5 % en poids, dans laquelle ledit ester d'acide oxyisobutyrique est choisi parmi un α-alcoxyisobutyrate d'alkyle représenté par la formule (I) : 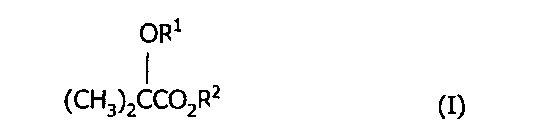 un β-alcoxyisobutyrate d'alkyle représenté par la formule (II) : 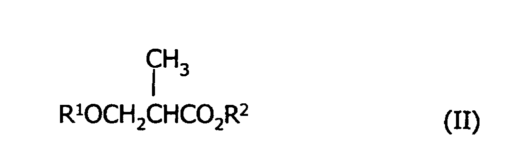 et un α-hydroxyisobutyrate d'alkyle représenté par la formule (III) : 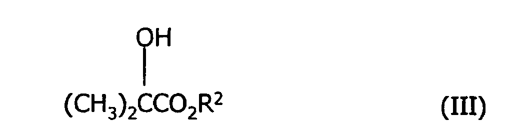 où R¹ et R² représentent chacun un groupe alkyle ayant de 1 à 4 atomes de carbone, comme solvant dans des revêtements, des additifs et des encres d'impression.

2. Utilisation selon la revendication 1, dans laquelle les revêtements, les adhésifs et les encres d'impression comprennent au moins un des constituants suivants : une résine de cellulose, une résine époxy, une résine acrylique, une résine vinylique, une résine alkyde, une résine de polyester, un polystyrène, un copolymère de méthacrylate de méthyle-styrène, un copolymère d'acrylonitrile-styrène, une résine phénoxy, une polysulfone.

3. Utilisation selon la revendication 1 ou 2, dans laquelle ladite composition de solvant comprend en outre au moins un composé choisi parmi l'eau, le méthanol, l'éthanol, l'alcool isopropylique, le butanol, le méthylisobutylcarbinol, l'heptanol, l'octanol, le nonanol, le 3-méthylbutanol, le propylèneglycol, le 3-méthoxybutanol, le 3-méthyl-3-méthoxybutanol, le 3,5,5-triméthyl-1-hexanol, le 2-éthyl-1-hexanol, le cyclohexanol, l'alcool benzylique, l'acétone, la méthyléthylcétone, la méthylisobutylcétone, la cyclohexanone, l'isophorone, la pyrrolidone, la N-méthylpyrrolidone, le diméthylformamide, le diméthylacétamide, le diméthylsulfoxyde, le sulfolane, l'acétate de méthyle, l'acétate d'éthyle, l'acétate de butyle, l'acétate d'amyle, l'acétate de 3-méthoxybutyle, l'acétate de 3-méthyl-3-méthoxybutyle, le lactate de méthyle, le lactate d'éthyle, le lactate de butyle, le 3-méthoxypropionate de méthyle, le 3-éthoxypropionate d'éthyle, l'acétate de 2-éthylhexyle, l'acétate de cyclohexyle, l'acétate de benzyle, le dibenzyléther, le nitrométhane, le nitroéthane, l'acétonitrile, la γ-butyrolactone, l'acétate de monométhyléther de propylèneglycol, le toluène, le xylène, l'hexane et le cyclohexane.

4. Utilisation selon l'une quelconque des revendications 1 à 3, dans laquelle ledit ester d'acide oxyisobutyrique est choisi parmi l'α-méthoxyisobutyrate de méthyle, l'α-méthoxyisobutyrate d'éthyle, l'α-éthoxyisobutyrate de méthyle, l'α-éthoxyisobutyrate d'éthyle, le β-méthoxyisobutyrate de méthyle, le β-méthoxyisobutyrate d'éthyle, le β-éthoxyisobutyrate de méthyle, le β-éthoxyisobutyrate d'éthyle, le β-isopropoxyisobutyrate de méthyle, le β-isopropoxyisobutyrate d'éthyle, le β-isopropoxyisobutyrate de butyle, le β-butoxyisobutyrate de méthyle, le β-butoxyisobutyrate d'éthyle, le β-butoxyisobutyrate de butyle, l'α-hydroxyisobutyrate de méthyle, l'α-hydroxyisobutyrate d'éthyle, l'α-hydroxyisobutyrate d'isopropyle et l'α-hydroxyisobutyrate de butyle.
